# EUROPEAN PATENT APPLICATION

(11) **EP 2 811 034 A1**
(43) Date of publication of application: **10.12.2014**
(21) Application number: 13761959.9
(22) Date of filing: 13.03.2013
(51) Int. Cl.: C12Q 1/37, A61K 31/7042, A61K 33/24, A61K 35/00, A61K 45/00, A61P 35/00, A61P 35/02, C12Q 1/02, G01N 33/15

(54) **METHOD FOR SCREENING ANTICANCER DRUGS, AND ANTICANCER DRUG THAT INDUCES CELL DEATH AND USES SUBSTANCE THAT INCREASES GRANZYME M ACTIVITY AS ACTIVE INGREDIENT**

(30) Priority: 14.03.2012 JP 2012057707
(71) Applicant: LSIP, LLC, Chiyoda-ku Tokyo 100-0005 (JP); Kubota, Shunichiro, Hino-shi, Tokyo 191-0033 (JP)
(72) Inventor: KUBOTA, Shunichiro, Hino-shi Tokyo 191-0033 (JP); SATO, Motohiko, Urayasu-shi Chiba 279-0014 (JP); WANG, Liyun, Tokyo 156-0052 (JP)
(74) Representative: Cabinet Plasseraud
(86) International application number: PCT/JP2013/057032
(87) International publication number: WO 2013/137328

(57) **Abstract**

The purpose of the present invention is to provide a method for screening anticancer drugs that use novel treatment mechanisms, and an anticancer drug that induces cell death and uses a substance that increases the activity of granzyme M as an active ingredient. This method for screening anticancer drugs comprises: a step for administering test substances to cancer cells; a step for detecting the activity of granzyme M in which cancer cells are expressed, and selecting test substances for which an increase in activity has been verified; and/or a step for detecting the presence or absence of cancer cell death induced by the activation of granzyme M in which cancer cells are expressed, and selecting test substances for which cell death has been verified.

## Description

### TECHNICAL FIELD

The present invention relates to a method for screening anticancer drugs, and an anticancer drug that induces cell death with a substance that increases granzyme M activity as an active ingredient.

### BACKGROUND ART

Cancer is one of the three largest causes of death in many countries, and thus the development of novel cancer curative drugs has been desired socially. Thus far, many anticancer drugs have been developed, and contributed to cancer treatment.

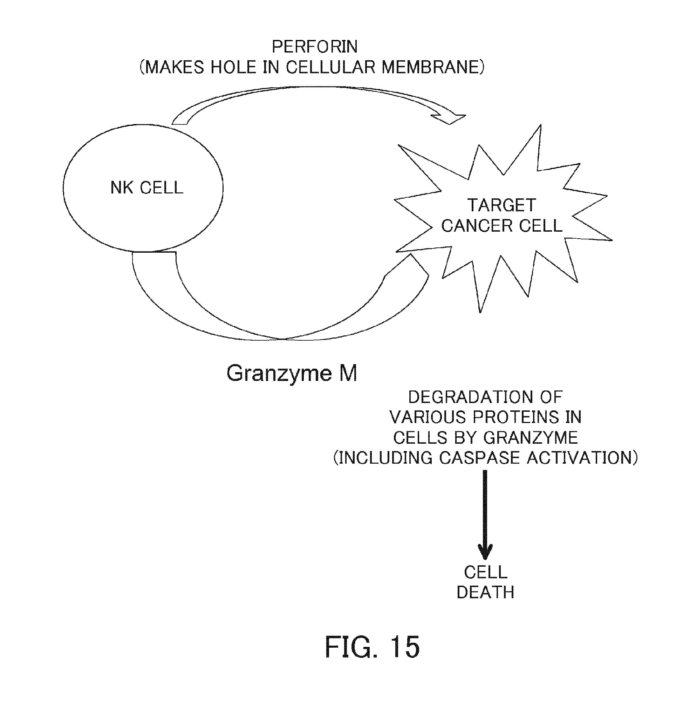

As an *in vivo* mechanism already established in the field of immunology, the mechanism of natural killer (NK) cells or cytotoxic T cells attacking cancer cells or virus-infected cells to induce cell death can be exemplified. With this mechanism, first, the NK cells or cytotoxic T cells secrete perforin to make holes in the cell membrane of the cancer cell or virus-infected cell, and in this state, secrete granzyme to penetrate inside the cell, and breakdown various intracellular proteins, thereby inducing cell death (refer to FIG. 15).

Granzyme is a serine protease, and the five types of A, B, H, K and M have been known to exist in humans (Non-patent Document 1). Thereamong, granzyme B is expressed in bladder cancer cells, and is being shown to participate in the permeation of cancer by breaking down the extracellular matrix of surrounding cells (Non-patent Document 2).

[Non-Patent Document 1] C.L. Ewen, K.P. Kane, and R.C. Bleackley, Review. A quarter century of granzymes, Cell Death and Differentiation. Advance Online Publication, 4 November 2011

[Non-Patent Document 2] D'Eliseo D., Pisu P., Romano C., Tubaro A., De Nunzio C., Morrone S., Santoni A., Stoppacciaro A., Velotti F., Granzyme B is expressed in urothelial carcinoma and promotes cancer cell invasion, Int. J. Cancer, 127, 1283-1294(2010)

### DISCLOSURE OF THE INVENTION

### Problems to be Solved by the Invention

However, the types of cancer are diverse, and the development of novel anticancer drugs is essential from hereon after. Then, in order to develop novel anticancer drugs, a screening method using an unconventional treatment mechanism is necessary.

The present invention takes account of the above situation, and has an object of providing a method for screening anticancer drugs using a novel treatment mechanism, as well as an anticancer drug that induces cell death with a substance that increases granzyme M activity as an active ingredient.

### Means for Solving the Problems

The present inventors have found that granzyme M, which had been considered to only exist in NK cells and cytotoxic T cells, is unexpectedly expressed with activity in human hematological cancer cells (cultured leukemia cells). Furthermore, it has been clarified that AraC activates granzyme M, thereby degrading NPM (nucleophosmin), which is a cell survival factor, to induce cell death of leukemia cells (U937 and HL60). In other words, the present inventors found that, when granzyme M is activated, cell death (apoptosis) of cancer cells is induced, thereby arriving at completing the present invention. More specifically, the present invention provides the following.

According to a first aspect, a method for screening anticancer drugs includes: a step of administering test substances to cancer cells; and a step of detecting the activity of granzyme M expressed in the cancer cells, and selecting the test substance for which an increase in the activity was confirmed.

According to a second aspect, a method for screening anticancer drugs includes: a step of administering test substances to cancer cells; and a step of detecting the presence of cell death of the cancer cells induced by activation of granzyme M expressed in the cancer cells, and selecting the test substance for which the cell death was confirmed.

According to a third aspect, in the method as described in the first or second aspect, the cancer cells are solid carcinoma cells or hematological cancer cells.

According to a fourth aspect, in the method as described in any one of the first to third aspects, perforin is not administered to the cancer cells.

According to a fifth aspect, in the method as described in any one of the first to fourth aspects, the test substance is a low molecular compound.

According to a sixth aspect, an anticancer drug that induces cell death includes a substance that increases the activity of granzyme M as an active ingredient.

According to a seventh aspect, in the anticancer drug as described in the sixth aspect, the substance increasing the activity of granzyme M is AraC, and induces cell death of hematological cancer cells.

According to an eighth aspect, in the anticancer drug as described in the seventh aspect, the hematological cancer is leukemia.

According to a ninth aspect, in the anticancer drug as described in the sixth aspect, the substance increasing the activity of granzyme M is cisplatin, and induces cell death of solid carcinoma cells.

According to a tenth aspect, in the anticancer drug as described in the ninth aspect, the solid carcinoma is cervical cancer.

### Effects of the Invention

According to the present invention, a method for screening anticancer drugs using a novel treatment mechanism, and an anticancer drug inducing cell death, with a substance that increases granzyme M activity as an active ingredient are provided.

### BRIEF DESCRIPTION OF THE DRAWINGS

FIG. 1 is a diagram showing the mechanism by which it is assumed that candidate substances obtained by the screening method according to the present invention treats or prevents cancer;
FIG. 2 gives the results of investigating the influence of AraC on the cell growth inhibition (cell death) of cancer cells (leukemia cells) (A), and a photograph showing the existence or non-existence of apoptosis associated proteins in cancer cells (leukemia cells, U937 cells and HL60 cells) by administration of AraC;
FIG. 3 provides photographs from investigating the influence by AraC on cell survival factor (anti-apoptotic factor, NPM) of cancer cells (leukemia cells), and a diagram showing the positions of fragments of cell survival factor (anti-apoptotic factor);
FIG. 4 is a photograph showing the expression level of granzyme M in cancer cells (leukemia cells, U937 cells);
FIG. 5 is a photograph showing the expression level of perforin in cancer cells (leukemia cells, U937 cells);
FIG. 6 is a photograph showing the expression levels of granzyme M and perforin in cancer cells (leukemia cells, HL60 cells) ;
FIG. 7 is a graph showing the relationship between administration of AraC and the activity of granzyme M in cancer cells;
FIG. 8 is a graph showing the relationship between administration of serine protease inhibitor (TPCK), and granzyme M activity in cancer cells (leukemia cells) via the administration of AraC;
FIG. 9 is a graph showing the relationship between the administration of a granzyme M-specific inhibitor, and the viable cell count of cancer cells (leukemia cells, HL60 cells) via the administration of AraC;
FIG. 10 is a graph showing the relationship between the administration of a granzyme M-specific inhibitor, and the viable cell count of cancer cells (leukemia cells, U937 cells) via the administration of AraC;
FIG. 11 is a graph showing the relationship between the administration of CDDP, and the activity of granzyme M in cancer cells (solid carcinoma, Hela cells);
FIG. 12 is a graph showing the relationship between the administration of a granzyme M-specific inhibitor, and the viable cell count of cancer cells (solid carcinoma, Hela cells) via the administration of CDDP;
FIG. 13 is a graph showing the activity of granzyme M in various cancer cells (KYSE180, KYSE450, HT29, HMY-1, A549 and LK2);
FIG. 14 provides photographs showing the localization of granzyme M in cancer cells (U937 and Hela cells);
FIG. 15 is a diagram showing the mechanism of cell death of cancer cells established by conventional immunology.

### PREFERRED MODE FOR CARRYING OUT THE INVENTION

Hereinafter, an embodiment of the present invention will be explained; however, the present invention is not to be limited to these.

### (Administration)

With the method for screening candidate substances for anticancer drugs according to the present invention, first, test substances are administered to cancer cells. The method of administration is not particularly limited so long as, for example, cultured cancer cells in a culture medium for cancer cells including the test substances (at least including the minimum nutrition for cancer cells)

As described next, the present invention is a screening method that does not introduce granzyme M from outside into the cancer cells, but rather focuses on granzyme M activity expressed in cancer cells.

### (Selection)

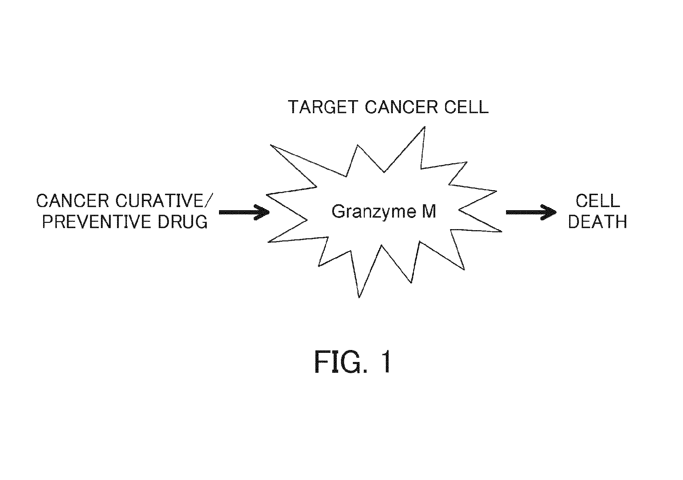

The present invention tries to obtain an anticancer drug that induces cell death of cancer cells, by activating granzyme M expressed in cancer cells, based on the novel finding in that granzyme M is expressed in cancer cells with activation (refer to FIG. 1). As a result thereof, the present invention includes a step of detecting the activity of granzyme M expressed in a cancer cell and selecting a test substance for which an increase in this activity has been confirmed, and/or a step of detecting the presence of cell death of cancer cells induced by activation of granzyme M expressed in the cancer cell, and selecting a test substance for which this cell death has been confirmed. It should be noted that cell death in the present invention is a broad concept encompassing apoptosis.

Granzyme M in itself is a well-known serine protease, and the activity and detection method thereof are well known as disclosed in Non-patent Document 1; Sami Mahrus, Walter Kisiel, and Charles S. Craik, Granzyme M is a regulatory protease that inactivates proteinase inhibitor 9, an endogenous inhibitor of granzyme B., J. Biol. Chem., 279, pp. 54275-54282, 2004; and the like. Although not particularly limited, the activity of granzyme M can be detected by arranging for peptides containing an amino acid sequence specifically cleaved by granzyme M (e.g., KVPL) to coexist with granzyme M, and detecting the fragments of peptide cleaved in the amino acid sequence thereof (e.g., electrophoresis method, and further, amino acid sequence analysis of fragments as necessary).

Although not particularly limited in the present invention, it is sufficient so long as recovering a protein group by a common method from cancer cells after the administration of the test substance ends (e.g., extraction), arranging for this protein group to coexist under an appropriate environment (pH, temperature, buffer, etc.) along with the peptide including the above-mentioned specifically cleaved amino acid sequence (e.g., KVPL), and detecting fragments of the peptide cleaved in this amino acid sequence. In this case, it is possible to specify a test substance for which an increase in granzyme M activity has been confirmed according to knowledge of peptide fragments being detected in large amounts and/or in a short time compared to a protein group of equivalent amount recovered from a control section (typically, a test section to which the test substance was not administered); and/or depending on the concentration of test substance; or the like.

Alternatively, the detection of granzyme M activity in cancer cells may be carried out, for a protein for which specifically being cleaved by granzyme M has been shown (e.g., anti-apoptotic factor such as nucleophosmin described later), among proteins expressed in cancer cells, by detecting fragments thereof in the protein group recovered from the cancer cells (e.g., Western analysis). Regarding nucleophosmin, it is possible to reference Cullen S. P., Afonina I. S., Donadini R., Luthi A. U., Medema J. P., Bird P. I., Martin S. J., Nucleophosmin is cleaved and inactivated by the cytotoxic granule protease granzyme M during natural killer cell-mediated killing, J. Biol. Chem., 284, 5137-5147, 2009. In this case, it is possible to specify a test substance for which an increase in granzyme M activity has been confirmed according to knowledge of peptide fragments being detected in large amounts compared to a protein group of equivalent amount recovered from a control section (typically, a test section to which the test substance was not administered); and/or depending on the concentration of test substance; or the like.

As described later, granzyme M activity in cancer cells does not always equal the expression level of granzyme M in cancer cells (transcription level in mRNA, synthesized amount of protein). In other words, even if the expression level of granzyme M in cancer cells does not increase, it is possible that the granzyme M activity in cancer cells increased. For this reason, from the viewpoint of broadly screening candidate substances for cancer curative or preventive drugs, it is more preferable to confirm the increase in granzyme M activity than an increase in expression level of granzyme M in cancer cells. However, this is not intended to exclude from the present invention the matter of jointly confirming the increase of granzyme M activity as well as an increase in the expression level of granzyme M in cancer cells.

From the viewpoint of raising screening accuracy, the matter of the existing amount of peptide fragments decreasing may be further confirmed by mixing a granzyme M inhibitor into the protein group recovered from cancer cells, as necessary. Depending on the extent of precision increase required, an inhibitor specific to granzyme M or a non-specific serine protease inhibitor can be used as the granzyme M inhibitor that can be used in this situation.

Regarding the cell death of cancer cells induced by activation of granzyme M, first, the presence of cell death of cancer cells can be detected by, for example, the matter of the growth rate of cancer cells cultured in a culture medium including the test substance (e.g., based on the dehydrogenase activity of cells) being low compared to a control section, and/or depending on the concentration of the test substance, etc. In addition, regarding this cell death being induced by the activation of granzyme M, for example, it may be carried out by detecting the matter of cell death being suppressed by the administration of an inhibitor specific to granzyme M being administered to cancer cells (typically, performing prior to or simultaneously with administration of test substance), and/or by detecting, for a protein specifically cleaved by granzyme M (e.g., anti-apoptotic factor such as nucleophosmin described later) among the proteins participating in cell death expressed in cancer cells, a fragment thereof in a protein group recovered from cancer cells (e.g., Western analysis).

It should be noted that, from the viewpoint of raising the screening accuracy, the test substance may be selected based on the increase in expression level/activity of granzyme M in cancer cells as necessary, in addition to cell death of the cancer cells induced by the activation of granzyme M.

### (Cancer Cell)

The cancer cells used are not particularly limited so long as expressing granzyme M. Generally classified, cancers are categorized into hematological cancers (leukemia, etc.) and solid carcinomas, and cancer cells corresponding to each type of cancer are conventionally known. As described later, among hematological cancer cells, in representative B cell-type cancer cells like U937 cells and HL60 cells; and in representative solid carcinoma cells like Hela cells, KYSE cells (esophageal cancer), HT29 cells (colorectal cancer), HMY-1 cells (melanoma), A549 cells (lung cancer) and LK2 cells (lung cancer), it has been proven by the present inventors that granzyme M is expressed with a low expression level, and the matter of T cell- and NK cell-type cancer cells expressing granzyme M is common technical knowledge. Therefore, many cancer cells are considered to be applicable in the present invention. It should be noted that KYSE180 and KYSE450 cells described later were provided by Prof. Yutaka Shimada of the University of Toyama, Department of Surgery and Science. HT29 cells, HMY-1 cells, A549 cells and LK2 cells were purchased from the Health Science Research Resources Bank (Rinku-Minamihama 2-11, Sennan-shi, Osaka).

Hematological cancer is a disease arising from cells included in the blood (erythrocyte, leukocyte, platelet) becoming cancerous, and leukemia, malignant lymphoma and multiple myeloma are representative examples thereof. Leukemia is a disease arising from hematopoietic stem cells in the bone marrow becoming cancerous, and is classified in the four types of acute myelocytic leukemia, chronic myelocytic leukemia, acute lymphocytic leukemia and chronic lymphocytic leukemia. Malignant lymphoma is a disease in which lymphocyte becomes malignant, and Hodgkin's lymphoma, non-Hodgkin's lymphoma (general term for B cell tumors and T/NK cell tumors other than Hodgkin's lymphoma), etc. are representative examples. Multiple myeloma is a disease arising from plasma cells in bone marrow (cells producing antibody) becoming cancerous.

From the viewpoint of raising the efficiency at which a promising cancer curative or preventive drug is obtained, it is preferable to use cancer cells corresponding to the type of cancer that is the target of the cancer curative or preventive drug. In addition, the cancer cells are preferably derived from a mammal, and more preferably derived from the same mammal as the animal to which the cancer curative or preventive drug will be administered. Normally, human derived cancer cells are preferable. However, the above-mentioned explanation has the intent of raising the efficiency of screening, and not the intent of excluding using candidate substances as curative drugs for cancers not corresponding with the cancer cells used.

### (Test Substance)

The test substance is not particularly limited, and may be any substance such as a natural or synthetic organic or inorganic low molecular or macromolecular substance. Due to focusing on a different mechanism from conventional screening methods, the present invention can use both a substance determined as a positive and a substance determined as a negative by a conventional screening method as the test substance.

The candidate substances selected in the present invention have a function of regulating the activity of granzyme M expressed in cancer cells, irrespective of the presence of perforin. For this reason, a promising test substance as a novel candidate substance is considered to be a substance that can permeate the cellular membrane of the cancer cell, ideally a low molecular compound, irrespective of the existence or non-existence of holes in the cellular membrane of the cancer cell. Therefore, it is preferable to use a low molecular compound as the test substance from the viewpoint of screening efficiency. However, there is also the possibility of the regulation of the activity of granzyme M expressed in the cancer cells being made through the binding or the like to a receptor, etc. presented on the cell surface of the cancer cell, and thus a high molecular substance can also be used as the test substance.

### (Anticancer Drug)

The present invention also encompasses anticancer drugs inducing cell death with a substance that increases the activity of granzyme M as the active ingredient. More specifically, although the anticancer drug according to the present invention is not particularly limited, it consists of one, two or more candidate compounds selected by way of the above-mentioned screening method (further screening may be performed as necessary), for example, AraC, cisplatin (CDDP), etc. More specifically, anticancer drugs inducing cell death of hematological cancer (i.e. leukemia) cells in which the active ingredient is AraC, and anticancer drugs inducing cell death of solid carcinoma (i.e. cervical cancer) cells in which the active ingredient is CDDP can be exemplified.

The anticancer drug of the present invention can be prepared in various drug formulations, and systemically or locally administered orally or parenterally. In the case of orally administering the present drug, the present drug may be formulated as a tablet, capsule, pellet, powdered medicine, pill, intravenous solution, suspension, emulsion, syrup, etc., or made a dry product that is re-dissolved upon use. In addition, in the case of parenterally administering the present drug, the present drug may be formulated as an intravenous injection agent (including intravenous drip), intramuscular injection agent, intraperitoneal injection agent, hypodermic injection agent, suppository, intratumoral directly administered agent, etc., and in the case of being an injectable formulation, may be provided in a state of a unit dosage ampule or a multi-administration amount container.

These various formulations can be produced by conventional methods appropriately using an excipient, expander, binder, wetting agent, disintegrator, lubricant, surfactant, dispersing agent, buffering agent, preservative, solubilizing agent, antiseptic, taste and flavor corrigent, soothing agent, stabilizer, tonicity agent, etc. that are generally used in formulations.

The dosage of the anticancer drug of the present invention will differ according to the age of the administration subject, administration route, administration frequency, symptoms, dosage form, etc.; however, a therapeutically effective amount of protein or polypeptide (i.e. effective amount) is in the range of about 0.001 to 30 mg/kg bodyweight, preferably about 0.01 to 25 mg/kg bodyweight, more preferably about 0.1 to 20 mg/kg bodyweight, even more preferably in the range of about 1 to 10 mg/kg, 2 to 9 mg/kg, 3 to 8 mg/kg, 4 to 7 mg/kg or 5 to 6 mg/kg bodyweight, and is administered from once per day to dividing into several times for one or more days. In addition, in the case of introducing polynucleotides coding proteins by way of a method such as gene therapy, it is sufficient so long as administering a polynucleotide that can express the protein in the above-mentioned quantity ranges.

### EXAMPLES

### Test Example 1

Using U937 cells and HL60 cells, which are human leukemia cells, and Colo201, which are human colon cancer cells (all purchased from cell bank), 3x10³ of each cell were seeded in 96 well plates, and then cultured with 100 µl of RPMI cell medium (containing 10% fetal bovine serum (FBS), 2 mM L-glutamine, 100 units/ml penicillin, 100 µg/ml streptomycin, 250 ng/ml amphoterin B) per each well. At this time, AraC (cylocide) being used in the treatment of acute myelocytic leukemia by conventional pathology was added to the culture medium at the respective concentrations of 1, 5, 10 and 50 µm, and cultured under the conditions of 37°C and 5% CO₂ for 24 hours. Subsequently, 10 µl of WST-8 solution was placed per 1 well, and cultured at 37°C for 4 hours.

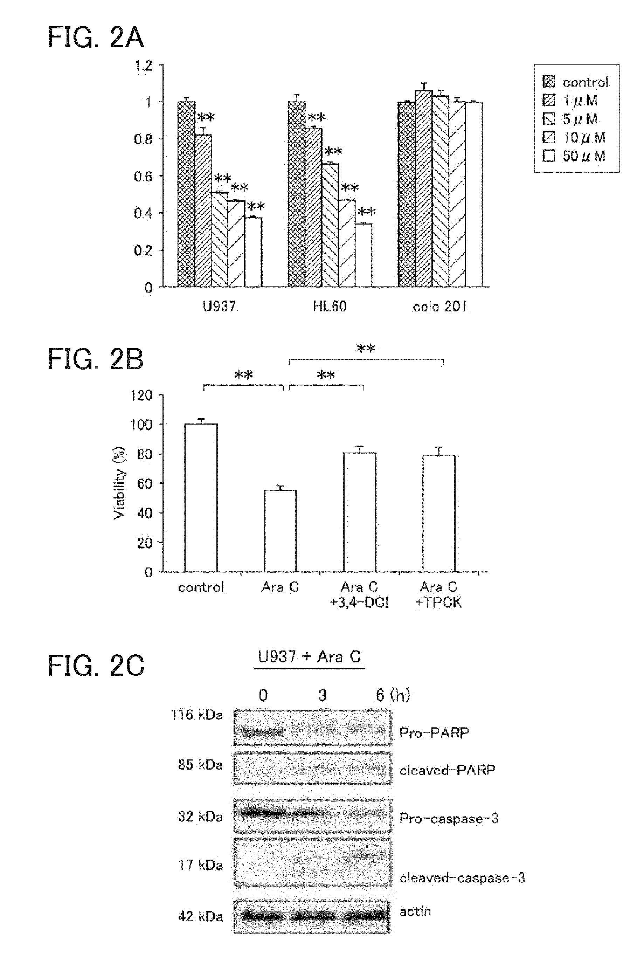

The growth of cells was measured using a Cell Counting Kit 8 (Dojindo). This method is a measurement method establishing the dehydrogenase activity in viable cells as an index, and performs measurement of viable cell count based on WST-8 (2-(2-methoxy-4-nitrophenyl)-3-(4-nitrophenyl)-5-(2,4-disulfophenyl)-2H-tetrazolium, monosodium salt), which is a tetrazolium salt, being reduced to form formazan. More specifically, using a spectrophotometer Multiscan JX (Thermo Labsystems, Waltham, MA), the absorbance at 450 nm was measured, and the absorbance at 690 nm was used as the reference. The results thereof are shown in FIG. 2A. The vertical axis in the graph of FIG. 2A represents the extent of cell viability by relative value.

As shown in FIG. 2A, cell death of U937 and HL60 was concentration dependently induced by AraC (**p<0.01). It should be noted that "**" in any of the drawings indicates p<0.01, and "*" indicates p<0.05. On the other hand, the cell death of Colo201, which is a colon cancer cell for which the therapeutic efficacy of AraC is not known, was not induced by AraC.

To the above-mentioned culture medium, a well-known serine protease inhibitor (50 µM TPCK, 5 µM 3,4-DCI) was added along with 5 µM of AraC, and it was analyzed whether cell death by AraC recovered. The results thereof are shown in FIG. 2B. The vertical axis in FIG. 2B represents the survival rate of cells by relative value.

As shown in FIG. 2B, cell death recovered significantly by the addition of serine protease inhibitor (**P<0.01). The participation of serine protease in the cell death of cancer cells by way of AraC is thereby suggested.

Immediately after culturing start (0 h) under the administration of 5 µM of AraC, after 3 hours and after 6 hours, the expression of apoptosis associated proteins of U937 cells was measured. The results thereof are shown in FIG. 2C.

As shown in FIG. 2C, PARP and caspase-3 are decomposed along with the elapse of time after AraC administration, whereby the participation of these in cell death was suggested. It thereby became clear that the cell death induced by AraC shown in FIGS. 2A and B is apoptosis.

### Test Example 2

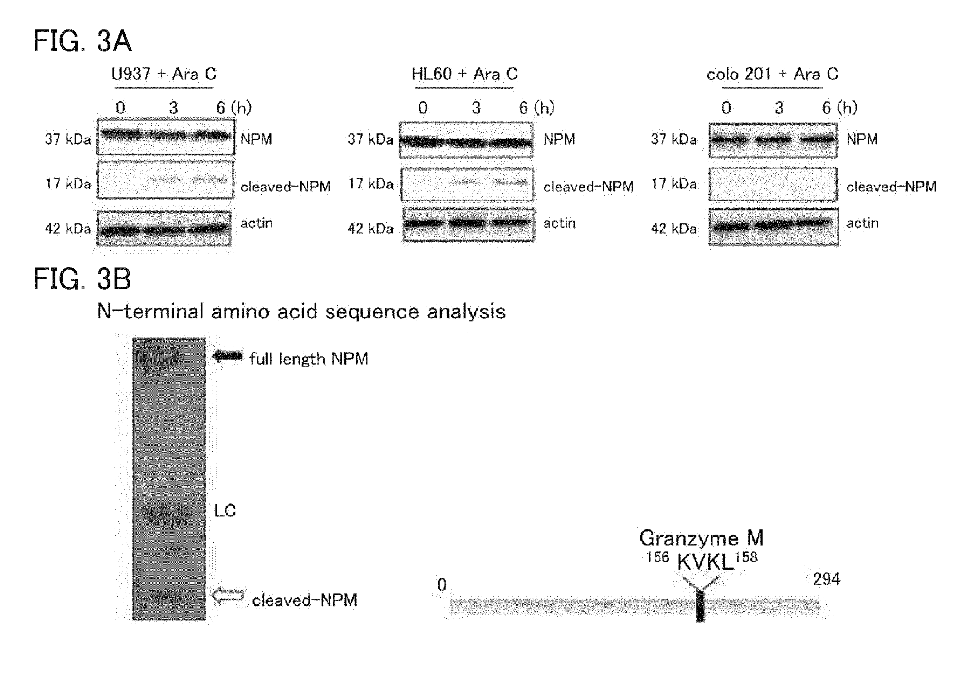

By the same method as FIG. 2C, protein groups extracted from the respective cells of U937, HL60 and Colo201 were molecular weight fractionated by way of electrophoresis of a conventional method, and the behavior of nucleophosmin (NPM), which is a cell survival factor (anti-apoptotic factor), was further investigated by the western blot method of a conventional method. It should be noted that monoclonal anti-GB23 (nucleophosmin/NPM) (catalog number B0556), which is an anti-NPM antibody, was purchased from Sigma-Aldrich (Saint Louis, Missouri, USA). The results thereof are shown in FIG. 3A.

As shown in FIG. 3A, for U937 and HL60, although fragments of NPM were detected along with the elapse of time after AraC administration, it was not detected for Colo201. It was thereby considered that the matter of inhibiting the function of cell survival factor (anti-apoptotic factor) is the mechanism of cell death of cancer cells induced by AraC.

NPM fragments were recovered by the immunoprecipitation method, and the N-terminal amino acids at the cleavage site of NPM were analyzed to carry out identification of the protease participating in the cleavage of NPM. The results thereof are shown in FIG. 3B. It should be noted that "LC" in FIG. 3B is a light chain of the antibody used in immunoprecipitation.

As shown in FIG. 3B, the N-terminal amino acid sequence of the fragment is AAED, whereby it was confirmed as being cleaved by Leu-158 of NPM. Due to the enzyme causing cleavage at this site being granzyme M (Cullen S. P., Afonina I. S., Donadini R., Luthi A. U., Medema J. P., Bird P. I., Martin S. J., Nucleophosmin is cleaved and inactivated by the cytotoxic granule protease granzyme M during natural killer cell-mediated killing, J. Biol. Chem., 284, pp. 5137-5147, 2009), the cleavage of NPM by AraC administration was considered to be from activation of granzyme M. It should be noted that, although not illustrated, similar results were also obtained for protein groups obtained from HL60 cells.

### Test Example 3

Using U937 cells, Colo201 cells (negative control) and KHYG-1 cells (positive control), which are human NK cells, 5 µM of AraC was added to the culture medium, and culturing was carried out. Immediately after culturing initiation (0 h), after 1 hour, after 3 hours and after 5 hours, cells were recovered from the cell medium, and protein groups were extracted from the cells. The amount of protein group was measured, and a certain amount of protein group was molecular weight fractionated by way of electrophoresis of a conventional method.

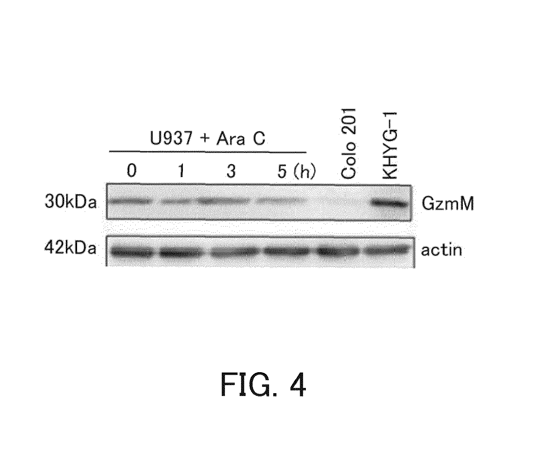

By way of western blot of a conventional method, the expression of granzyme M was detected. The results thereof are shown in FIG. 4. As shown in FIG. 4, it was found that granzyme M is expressed in U937 cells. The expression of granzyme M in U937 cells, which are cancer cells, is a novel discovery that had not been known conventionally. It should be noted that the expression level of granzyme M in U937 cells was unrelated to the elapse of AraC administration time. It should be noted that GZMM Antibody (catalog number: AP6792c) by Abgent was used as the granzyme M antibody.

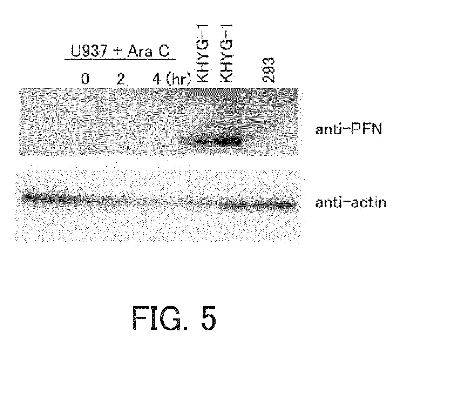

The expression of perforin was detected by way of western blot of a conventional method. The results thereof are shown in FIG. 5. As shown in FIG. 5, it was confirmed that the expression of perforin was not induced by AraC in U937 cells. It should be noted that Mouse Anti-Human Perforin, Monoclonal Antibody (catalog number: 3465-6-250) by Mabtech was used as the perforin antibody.

Therefore, the fragments of NPM from AraC administration were from granzyme M expressed in U937 cells; however, it was considered to be unrelated to the induced expression of perforin.

### Test Example 4

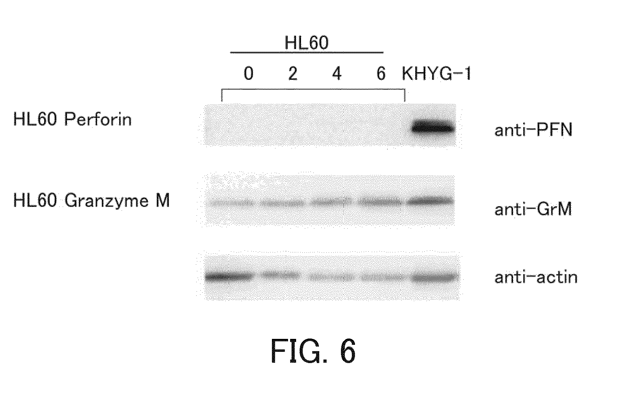

Except for the point of using HL60 cells and KHYG-1 cells (positive control section), the expression of granzyme M and perforin was examined at the same conditions as Test Example 3. The results thereof are shown in FIG. 6. As shown in FIG. 6, it was confirmed also for HL60 cells that, although the expression of perforin was not recognized, granzyme M was expressed. It should be noted that granzyme M in HL60 cells showed a tendency of the expression level being exacerbated depending on the time elapsed after AraC administration.

Therefore, it was suggested that the cleavage of NPM from AraC administration is due to activation of granzyme M expressed in HL60 cells, but the conventionally known perforin does not participate.

### Test Example 5

At the same conditions (5 µM AraC) as Test Example 1, U937 cells, HL60 cells and KHYG-1 cells (positive control section) were cultured, cells were recovered from the culture medium immediately after culturing initiation (0 h), after 3 hours, after 5 hours and after 7 hours, and the protein groups were extracted from the cells.

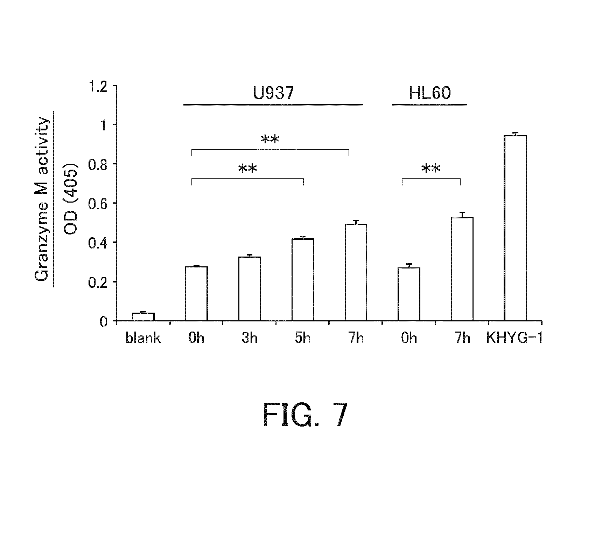

To a measurement buffer (100 mM HEPES, 50 mM CaCl₂, pH 7.4), 30 µg of protein group was introduced, and after adding substrate in an amount for a concentration of 200 µM, was incubated at 37°C for 1 hour, and the absorbance at OD 405 nm was measured. It should be noted that the substrate was one synthesized by referencing Sami Mahrus, Walter Kisiel, and Charles S. Craik, Granzyme M is a regulatory protease that inactivates proteinase inhibitor 9, an endogenous inhibitor of granzyme B, J. Biol. Chem., 279, pp. 54275-54282, 2004. The results thereof are shown in FIG. 7. The vertical axis in FIG. 7 is an activity relative value for granzyme M calculated based on the absorbance.

As shown in FIG. 7, it was found that the activity of granzyme M in U937 cells and HL60 cells increases along with the elapse of time after AraC administration.

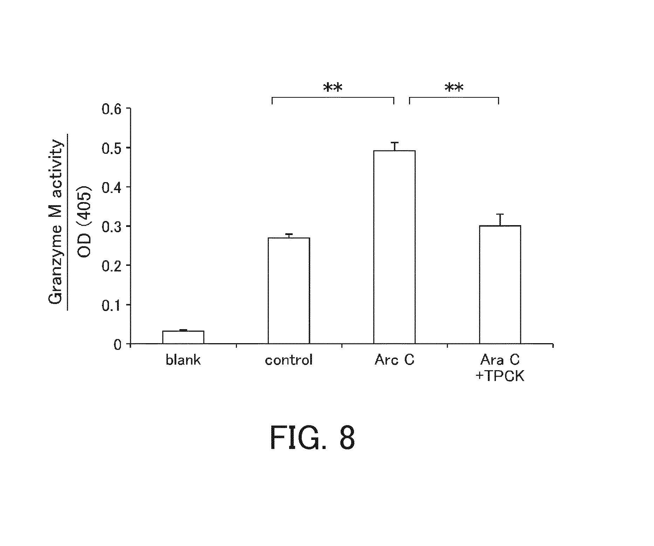

Next, using protein groups arrived at by administering serine protease inhibitor (50 µM TPCK) to the culture medium, culturing U937 cells under the same conditions for 7 hours, and recovering, the absorbance was measured in the same way as above. The results thereof are shown in FIG. 8. The vertical axis in FIG. 8 is the activity relative value for granzyme M calculated based on the absorbance.

As shown in FIG. 8, the activity increase in granzyme M in U937 cells from AraC administration was assumed to actually be granzyme M due to being significantly suppressed (**P<0.01) by serine protease inhibitor.

### Test Example 6

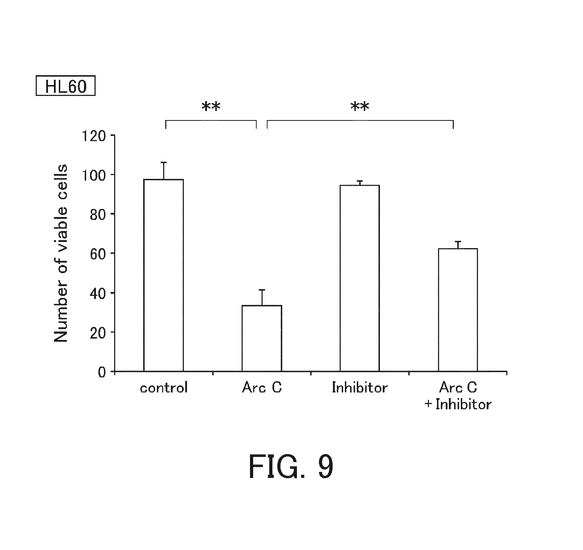

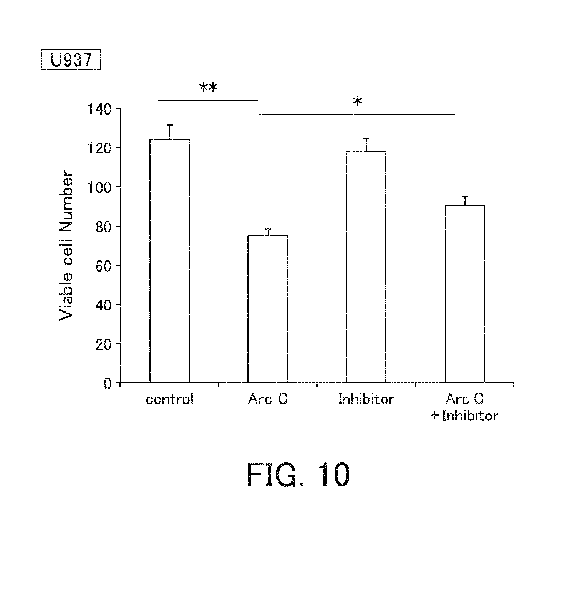

Ac-KVPL-CMK, which is an inhibitor specific to granzyme M, was added to the culture medium (5 µM AraC) in an amount making a final concentration of 0.75 mg/ml, and then preincubated. To 48 well plates, 5x10⁵ of each cell (U937 cells and HL60 cells) were seeded, and culturing was initiated with 200 µl of culture medium per well. Fourteen hours after culturing initiation, the number of viable cells was measured by the tryptan blue method (mixing a cell suspension and tryptan blue solution in 1:1, placing in a hemocytometer, and counting the number of cells using an optical microscope). It should be noted that Ac-KVPL-CMK was synthesized based on the tertiary structure of granzyme M (L. Wu et al., Structural basis for proteolytic specificity of the human apoptosis-inducing granzyme M, J. Immunol., 2009; 183: 421-429). The results thereof are shown in FIGS. 9 and 10.

As shown in FIGS. 9 and 10, significant recovery of the viable cell count (**P<0.01 for HL60 cells, *P<0.05 for U937 cells) compared to the group to which only AraC was administered was found in the group to which the granzyme M-specific inhibitor was administered along with Arac for both HL60 cells and U937 cells. It was thereby proven that the cell death of U937 cells and HL60 cells from AraC is induced by the activation of granzyme M. It should be noted that, since a significant difference in viable cell count was not confirmed between the sole administration of specific inhibitor group and the control group, it was confirmed that specific inhibitor is administered in a concentration not influencing the cell count alone.

### Test Example 7

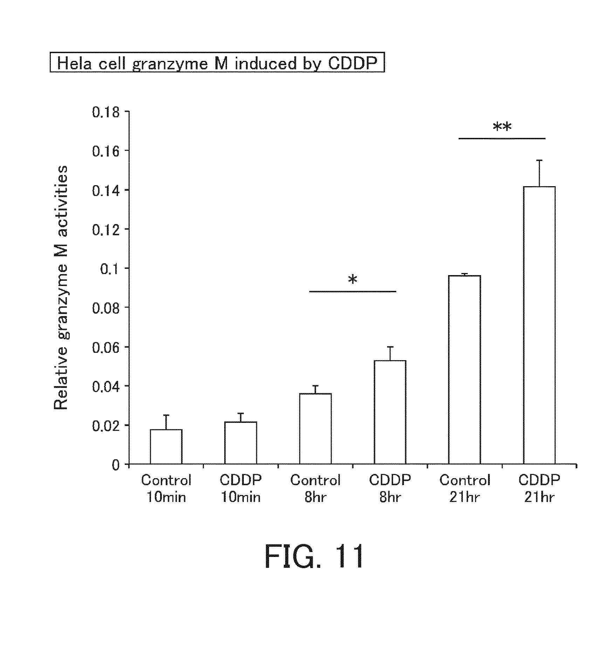

Using Hela cells, which are human solid carcinoma cells, the action causing granzyme M activity of CDDP 50 µM conventionally used as a curative drug for solid carcinoma was investigated by culturing Hela cells for 24 hours at the same conditions as Test Example 5. The results thereof are shown in FIG. 11. The vertical axis in FIG. 11 is the activity relative value for granzyme M calculated based on the absorbance, and on the horizontal axis are test groups having incubation times of "10 min", "8 hr" (8 hours) and "21 hr" (21 hours), in which "control" is the substrate without CDDP, and "CDDP" is the substrate and CDDP addition. In the test groups of 8 hr and 21 hr, a significant difference in each comparing with the control of each time (*P<0.05 for 8 hours, **P<0.01 for 21 hours) was recognized, whereby it was found that the activity of granzyme M increased with the elapse of time.

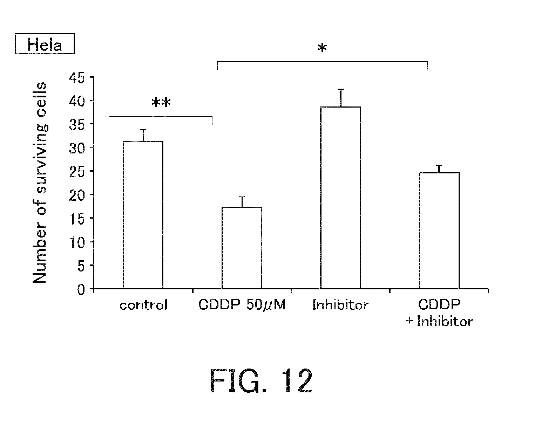

Hela cells were cultured for 24 hours to investigate the recovery of cell death from CDDP by way of the granzyme M-specific inhibitor. The results thereof are shown in FIG. 12. As shown in FIG. 12, also for Hela cells, a significant recovery in viable cell count (*P<0.05) compared to the group to which CDDP was administered alone was found in the group to which granzyme M-specific inhibitor was administered along with CDDP. It was thereby proven that the cell death of Hela cells from CDDP is also induced by the activation of granzyme

### M.

### Test Example 8

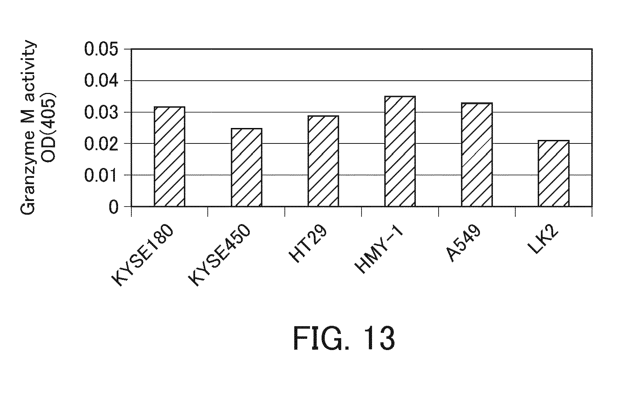

Using protein groups arrived at by culturing, KYSE180 cells, KYSE450 cells, HT29 cells, HMY-1 cells, A549 cells and LK2 cells for 7 hours at the conditions described in paragraph 0059, and then recovering, the absorbance was measured. The results thereof are shown in FIG. 13. The vertical axis in FIG. 13 is the activity relative value of granzyme M calculated based on the absorbance.

As shown in FIG. 13, although the expression level was low, the activity of granzyme M was confirmed for all cells. These results support the fact that granzyme M is expressed in a wide variety of cancer cells, and many cancer cells can be used in the present invention.

### Test Example 9

With U937 cells and Hela cells, experiments to clarify the presence of granzyme M (localization in cells) was performed (immunohistostaining using granzyme M-specific antibody).

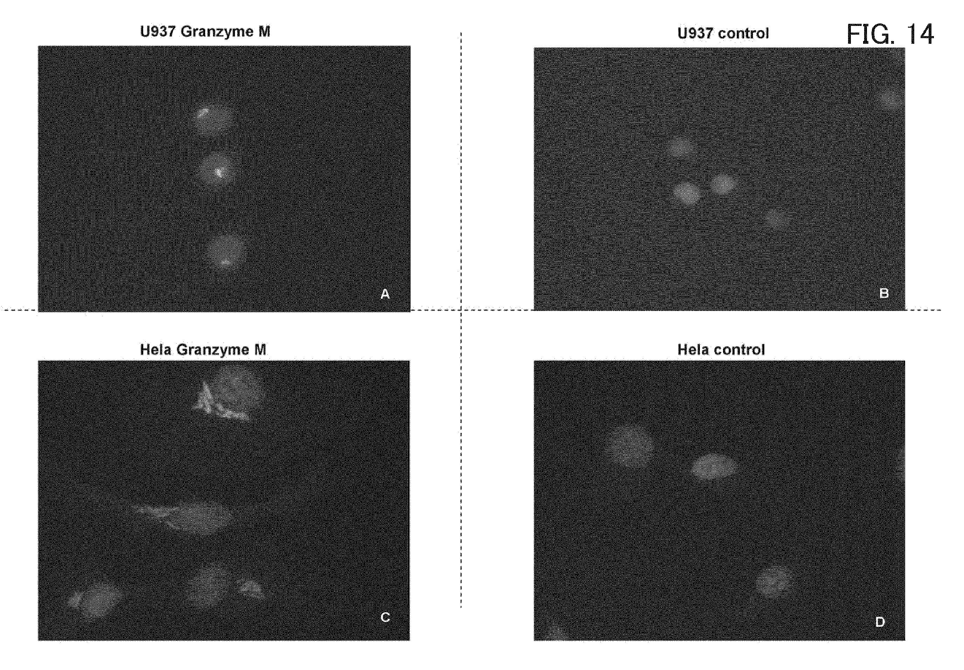

More specifically, after cold methanol treating U937 cells, blocking was performed with a PBS solution with 10% FBS as the blocking buffer. Subsequently, using granzyme M antibody (diluted 25 times with blocking buffer) as the primary antibody and anti-mouse IgG (H+L)-Alex488 (diluted 1,000 times with blocking buffer) as the secondary antibody, immunostaining was performed. In addition, as a control, it was stained with only anti-mouse IgG(H+L)-Alexa488 (diluted 1,000 times with blocking buffer). For both, the nucleus was immunostained using 200-fold diluted solution of DAPI solution (D523 manufactured by Dojindo). The results thereof are shown in FIGS. 14A and B, respectively. In addition, the results arrived at by performing the same treatment for Hela cells are shown in FIGS. 14C and D, respectively. It should be noted that, in FIG. 14, the portion appearing in strong white corresponds to Alexa 488 (i.e. granzyme M protein), and the portion appearing in weak white (grey) corresponds to the DAPI stained portion (i.e. nucleus), respectively.

As shown in FIG. 14, it was found that granzyme M protein localizes in cells in both U937 cells and Hela cells. This fact supports the mechanism of the cell death induction of cancer cells exerted by the present invention being due to the activation of granzyme M present in cancer cells.

## Claims

1. A method for screening anticancer drugs, comprising:
a step of administering test substances to cancer cells; and
a step of detecting the activity of granzyme M expressed in the cancer cells, and selecting the test substance for which an increase in the activity was confirmed.

2. A method for screening anticancer drugs, comprising:
a step of administering test substances to cancer cells; and
a step of detecting the presence of cell death of the cancer cells induced by activation of granzyme M expressed in the cancer cells, and selecting the test substance for which the cell death was confirmed.

3. The method according to claim 1 or 2, wherein the cancer cells are solid carcinoma cells or hematological cancer cells.

4. The method according to any one of claims 1 to 3, wherein perforin is not administered to the cancer cells.

5. The method according to any one of claims 1 to 4, wherein the test substance is a low molecular compound.

6. An anticancer drug that induces cell death, comprising a substance that increases the activity of granzyme M as an active ingredient.

7. The anticancer drug according to claim 6, wherein the substance increasing the activity of granzyme M is AraC, and induces cell death of hematological cancer cells.

8. The anticancer drug according to claim 7, wherein the hematological cancer is leukemia.

9. The anticancer drug according to claim 6, wherein the substance increasing the activity of granzyme M is cisplatin, and induces cell death of solid carcinoma cells.

10. The anticancer drug according to claim 9, wherein the solid carcinoma is cervical cancer.
